# EUROPEAN PATENT SPECIFICATION

(11) **EP 0 398 955 B1**
(45) Date of publication and mention of the grant of the patent: **08.07.1998**
(21) Application number: 89902154.7
(22) Date of filing: 23.01.1989
(51) Int. Cl.: C12Q 1/68, G01N 33/00

(54) **DIAGNOSIS OF RETINOBLASTOMA**
DIAGNOSE VON RETINOBLASTOMA
DIAGNOSTIC DU RETINOBLASTOME

(30) Priority: 21.01.1988 US 146525
(43) Date of publication of application: 28.11.1990
(73) Proprietor: MASSACHUSETTS EYE & EAR INFIRMARY, Boston, MA 02114 (US); WHITEHEAD INSTITUTE, Cambridge, MA 02130 (US)
(72) Inventor: Dryja, Thaddeus P., Milton, MA 02143 (US); Friend, Stephen, Sommerville, MA 02143 (US); Yandell, David W., Waltham, MA 02154 (US)
(74) Representative: Deans, Michael John Percy
(86) International application number: US8900293
(87) International publication number: WO8906703

(56) References cited:
- EP-?- 0 259 031
- EP-A- 0 293 266
- US---S- D16 8 9
- US---S- D16 8 9
- NATURE, vol. 323, 16th October 1986, pages 643-646, London, GB; S.H. FRIEND et al.: "A human DNA segment with properties of the gene that predisposes to retinoblastoma and osteosarcoma"
- SCIENCE, vol. 236, 26th June 1987, pages 1657-1661, Lancaster, PA, US; Y.-K.T. FUNG et al.: "Structural evidence for the authenticity of the human retinoblastoma gene"
- NATURE, vol. 329, no. 6140, 15th October 1987, pages 642-645, London, GB; W.-H. LEE et al.: "The retinoblastoma susceptibility gene encodes a nuclear phosphoprotein associated with DNA binding activity"
- Science, Volume 235 issued 13 March 1987 (Washington, D.C. USA) W. LEE "Human Retinoblastoma Susceptibility Gene: Cloning, Identification and Sequence" entire Article.
- CHEMICAL ABSTRACTS, Volume 107, No. 9 issued August 1987 (Columbus, Ohio, USA) S.A. LATT "DNA Based Detection of Chromosome Deletion & Amplification" Abstract No. 71579;, Cold Spring Harbor Symp. Quant. Biol. 51(1) 299-307
- CHEMICAL ABSTRACTS, Volume 106 No. 7, issued February 1987 (Columbus, Ohio, USA) H. SCHEFFER, "A Straightforward Approach to Isolate DNA Sequences with Potential Linkage to the Retinoblastoma Locus" Abstract No. 44946Z, Hum. Genet. 74(3) 249-255
- CHEMICAL ABSTRACTS, Volume 105, No. 17, issued October 1986 (Columbus, Ohio, USA) E.Y. LEE, "Molecular Cloning of the Human Esterase D Gene a Genetic Marker of Retinoblastoma" Abstract No. 147520q, Proc. Natl. Acad. Sci. U.S.A. 83(17) 6337-41.
- CHEMICAL ABSTRACTS, Volume 102, No. 7 issued February 1985 (Columbus, Ohio, U.S.A.) M. LALANDE "Isolation of Human Chromosome 13-Specific DNA Sequences Cloned from Flow Sorted Chromosome and Potentially Linked to the Retinoblastoma Locus" Abstract No. 57143C, Cancer Genet. Cytogenet 13(4) 283-95.

## Description

### Background of the Invention

This invention concerns the retinoblastoma gene and methods for detecting and treating patients afflicted with a defective retinoblastoma gene.

Retinoblastoma is a neoplastic condition of the retinal cells, observed almost exclusively in children between the ages of 0 and 4 years. It affects between 1 in 34,000 and 1 in 15,000 live births in the United States. (L.E. Zimmerman, 1985, Retinoblastoma and retinocytoma, In W.H. Spencer (ed.), Ophthalmic Pathology: an Atlas and Textbook, Vol. II, Philadelphia: W.B. Saunders Co., pp. 1292-1351.) If untreated, the malignant neoplastic retinal cells in the intraocular tumor travel to other parts of the body, forming foci of uncontrolled growth which are always fatal. The current treatment for a retinoblastoma is enucleation of the affected eye if the intraocular tumor is large; for small intraocular tumors, radiation therapy, laser therapy, or cryotherapy is preferred. There is no known successful treatment for metastatic retinoblastoma. As with most cancers, morbidity and mortality are reduced if diagnosis can be made early in the course of the disease.

In 30-40% of cases of retinoblastoma, the affected individual carries a heritable predisposition to retinoblastoma and can transmit this predisposition to his or her offspring as a dominant trait (A.G. Knudson, 1971, Mutation and cancer: Statistical study of retinoblastoma, Proc. Natl. Acad. Sci., Vol. 68, pp. 820-23). Carriers of this retinoblastoma-predisposing trait are at a greatly elevated risk for development of several other forms of primary cancer, notably osteosarcoma and soft-tissue sarcoma.

The genetic locus associated with familial retinoblastoma has been assigned to the q14 band of human chromosome 13 (R.S. Sparkes et al., 1980, Science, Vol 208, pp. 1042-44). Most retinoblastomas arise from cells which have )lost both normal, dominant, homologous alleles at this retinoblastoma locus. However, individuals carrying one defective allele may be predisposed to the disease. Children who have had one eye affected by retinoblastoma or who are related to someone with retinoblastoma may be genetically predisposed and therefore at risk of developing the disease. These individuals routinely are tested for retinoblastoma every 2-3 months by an ocular examination procedure which requires placing the child under general anesthesia.

EP-A-259031, published after the priority date of the present application discloses human DNA for use in the detection of retinoblastoma. Nature Vol. 323 (1986) pp 643-646 discloses a human DNA segment with properties of the gene that predisposes to retinoblastoma and osteosarcoma, the fragment detecting a locus which is frequently deleted in retinoblastomas and osteosarcomas. Science Vol. 236 (1987) pp 1657-1661 describes probing retinoblastomas with a cDNA shown to be the Rb gene for identifiable structural changes such as deletions producing truncated transcripts. Nature Vol. 329 (1987) p 642-645 discloses the characterisation of the retinoblastoma gene product as a nuclear phosphoprotein associated with DNA binding activity.

In general, the invention provides a method of diagnosing predisposition of a human patient to retinoblastoma, said method comprising the step of detecting, in the retinoblastoma gene of said patient, a genetic polymorphism selected from the group consisting of:
(a) a restriction fragment length polymorphism (RFLP) in the genomic retinoblastoma gene of said patient;
(b) an altered recognition sequence of a restriction endonuclease selected from the group consisting of RsaI, KpnI, XbaI, MboII, and Tth111I;
(c) variability in the number of tandem repeats of at least a substantial portion of the nucleic acid sequence shown in Table 2; and
(d) variability in the number of tandem repeats of the nucleic acid sequence GGGNNGTGGGG, where N is A, T, C, or G.

We describe methods of screening human patients to determine those not at risk of developing retinoblastoma and thus not requiring conventional examinations to be performed. This screening involves, for example, comparing nucleic acid of a patient with purified nucleic acid encoding a human Rb gene, or fragments thereof.

Methods of analysing the predisposition of patients to retinoblastoma are described which involve detecting large and small deletions or point mutations in the retinoblastoma gene, or detecting the co-inheritance of such defects with specific restriction fragment length polymorphisms (RFLPs), or detecting the presence or absence of a normal or defective retinoblastoma gene by hybridizing a nucleic acid sample from the patient with a probe specific for the retinoblastoma gene, and determining the ability of the probe to hybridize to the nucleic acid. The lack of hybridization to the nucleic acid indicates the presence of a large deletion in the gene. A probe specific for the retinoblastoma gene may be hybridized to fragments separated by a defined physical property from a sample. of a patient, the hybrids of the probe and the fragments detected, and the hybrids compared to hybrids detected from the hybridization of the probe and separated nucleic acid fragments from a normal retinoblastoma gene. The absence of hybrids or presence of hybrids of a smaller size compared to a normal patient is an indication of large deletions in the retinoblastoma gene of the patient. Preferably, the probe specific for the retinoblastoma gene is the cloned DNA in p4.7R, or a fragment thereof; and the defined physical property is molecular weight.

Small deletions or point mutations can be detected by determining the nucleotide sequence of a retinoblastoma allele from a patient, and comparing the nucleotide sequence with the nucleotide sequence of a retinoblastoma allele, or subregion thereof, from a person not afflicted with retinoblastoma; or by detecting mismatches between a nucleic acid sample from a patient and a probe specific for the retinoblastoma gene from a person not afflicted with retinoblastoma. The co-inheritance of specific genetic polymorphisms with the retinoblastoma gene may be an indication of the predisposition of a patient to retinoblastoma. According to this method, nucleic acid fragments are generated from a sample of the patient, the fragments are separated according to a defined physical property of the fragments (e.g., molecular weight), a detectable probe specific for the retinoblastoma gene is hybridized to the fragments, hybrids of the probe and the fragments are detected, and the hybrids are compared to hybrids detected from the hybridization of the same probe and separated nucleic acid fragments from a sample of a parent of the patient.

We also describe the use of an isolated normal human retinoblastoma gene to synthesize Rb polypeptide for use in the treatment of individuals determined to have a defective Rb allele.

A method of detecting the presence of the retinoblastoma polypeptide is also described in

a tumor sample from a human patient, by producing an antibody to the retinoblastoma polypeptide, contacting the antibody with the tumor sample, and detecting immune complexes as an indication of the presence in the tumor sample of the retinoblastoma polypeptide. The absence of the polypeptide indicates that the tumor is caused by a defect in a retinoblastoma allele. This procedure would preferably involve contacting a tumor sample from a human patient with an antibody (e.g., monoclonal antibody) which specifically reacts with the retinoblastoma polypeptide, or a fragment thereof, and determining whether the antibody binds to cells of the tissue specimens. The absence of immune complexes is an indication that the tumor was the result of a defective retinoblastoma allele.

Other features and advantages of the invention will be apparent from the following description of the preferred embodiments thereof, and from the claims.

### Description of the Preferred Embodiments

The description of the preferred embodiments of the invention will follow the brief description of the drawings given below.

### Drawings

Fig. 1 is a diagrammatic representation of a restriction map of the insert in the clone p4.7R.

Fig. 2 is a diagrammatic representation of a restriction map of the genomic locus of the retinoblastoma gene.

Fig. 3 is a diagrammatic representation of the vectors p2AR3.8 and p2AR0.9 of the invention.

Fig. 4 is a scale map of the normal retinoblastoma gene.

Fig. 5 (5-1 through 5-3) is a nucleic acid sequence of a cDNA of the normal retinoblastoma gene, with flanking regions.

Fig. 6 (6-1 through 6-9) is a nucleic acid sequence of exons of the normal retinoblastoma gene, with flanking regions.

Fig. 7 is a restriction map of the retinoblastoma gene, showing the locations of DSPs.

Fig. 8 is a gel and a diagram showing the inheritance og the polymorphism RB1.3 in a retinoblastoma-prone family.

Fig. 9 is a diagram showing the segregation of the DSP RB1.3 in three families with hereditary retinoblastoma.

### Retinoblastoma Polypeptide

The Rb polypeptide is the specific amino acid chain encoded by the nucleic acid sequence of the normal retinoblastoma gene. The Rb polypeptide of this invention includes: (1) naturally occurring retinoblastoma protein; (2) synthetically produced retinoblastoma polypeptide; and (3) retinoblastoma polypeptide produced from purified nucleic acid (e.g., cDNA or genomic DNA) via an in vitro expression system. Also included are biologically active fragments of Rb polypeptide which either have a biological activity of naturally occurring Rb polypeptide, or include an epitope of this polypeptide and thus are suitable for production of Rb-specific antibodies.

### Retinoblastoma Gene

The Rb gene is that distinct nucleic acid sequence in the human genome, the absence or mutation of which predisposes one to retinoblastoma. The purified nucleic acid sequence encoding the retinoblastoma gene can be carried on vectors which can be propagated in cells. For the purposes of this invention, purified nucleic acid encoding the Rb gene is defined as nucleic acid isolated from its natural environment (e.g., cDNA or a fragment of genomic DNA) which hybridizes specifically to the retinoblastoma gene under hybridizing conditions. An example of purified nucleic acid which encodes the retinoblastoma gene, and is carried on a vector, is the cDNA clone p4.7R. This clone was obtained in the following manner.

### cDNA

The human DNA probe pH3-8, isolated from a human chromosome 13 lambda phage library (M. Lalande et al., 1984, Cancer Genet. Cytogenet., Vol. 13, pp. 283-95), was used in a chromosome walking technique to isolate and map 30 kilobases (kb) of genomic DNA surrounding the H3-8 sequence. One fragment generated by this technique, named p7H30.7R, was found to recognize a DNA sequence in the mouse genome as well as one within human chromosome 13 (T.P. Dryja et al., 1986, Proc. Natl. Acad. Sci. USA, Vol. 83, pp. 7391-94). The homology of p7H30.7R to both human and mouse DNA suggested that p7H30.7R contains coding sequences of a structural gene.

To test this possibility, p7H30.7R was radiolabeled and used to probe a Northern blot of RNA isolated from three retinoblastoma tumors and an adenovirus 12-transformed human embryonic retinal cell line (Vaessen et al., 1986, EMBO Journal, Vol. 5, pp. 335-). The p7H30.7R probe hybridized to an RNA transcript of approximately 4.7 kb from the retinal cell line, but did not hybridize to any RNA transcripts from the three tumor samples.

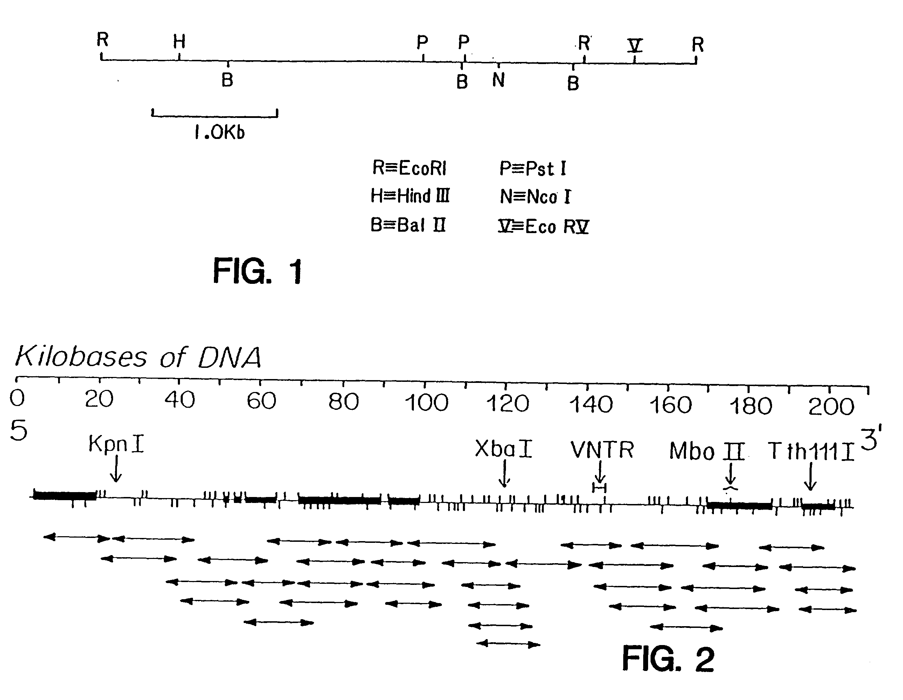

Subsequently, RNA isolated from the adenovirus-transformed retinal cell line was used to construct a cDNA library. This library was screened with the labeled p7H30.7R probe. Several cDNA clones were isolated which had similar restriction maps. The longest of these, p4.7R, contained 4.7 kb of DNA. The restriction map of the insert in the clone p4.7R is shown in Fig. 1.

The p4.7R clone was used to screen RNA transcripts isolated from four retinoblastomas, an osteosarcoma, and the adenovirus-transformed retinal cells. In a Northern blot analysis of isolated RNA's, the p4.7R probe cross-reacted with a 4.7 kb transcript in the transformed retinal cells which was not present in the four retinoblastoma and one osteosarcoma cell samples.

### Genomic DNA

Clones containing genomic DNA including the retinoblastoma gene were isolated in the manner described below. Recombinant bacteriophage libraries containing human genomic DNA fragments inserted in the lambda phage cloning vector EMBL-3 were constructed according to published methods (Seed et al., 1982, Gene, Vol. 19, pp. 201-209). Those recombinant bacteriophage which contain fragments of the retinoblastoma gene were initially detected by hybridization of the bacteriophage plaques with p4.7R.

Thirty-six distinct recombinant bacteriophage that contain overlapping human genomic DNA fragments were isolated. Selected bacteriophage were plaque-purified and amplified, and the restriction map of each phage insert was determined by the method of Rackwitz et al., Gene, Vol. 30, pp. 195-200.

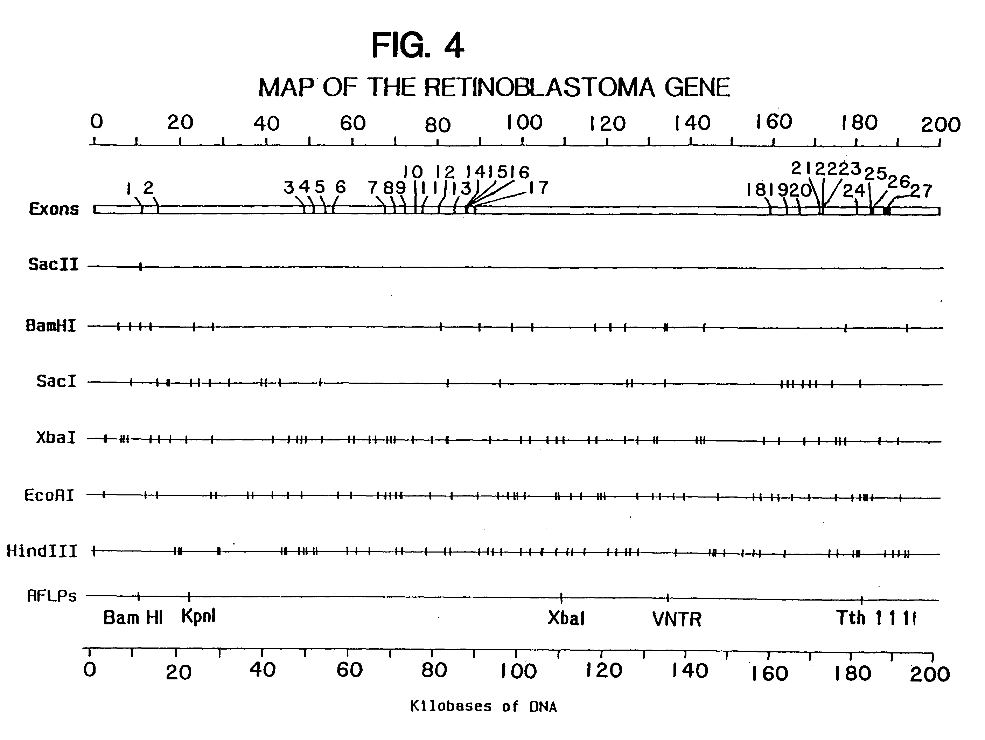

With these bacteriophage a restriction map of a region that spans approximately 200 kb was constructed, shown in Figs. 2 and 4. All of the known sequences present in the mRNA from the retinoblastoma gene are present in this cloned region. In aggregate, the human DNA sequences in this set of bacteriophage represent the chromosomal segment of the Rb gene. In Fig. 2, the vertical marks above the map represent the location of HindIII sites, and the vertical marks below the map represent the location of EcoRI sites. The boxed areas represent HindIII fragments which contain sequences found in the cDNA (exons). Each double-headed arrow beneath the map represents a distinct recombinant bacteriophage clone. Fig. 4 shows all the recognition sites of the six restriction enzymes Hind III, EcoR I, Xba I, Sac I, Sac II, and BamH I (New England Biolabs, Inc.). Restriction endonuclease fragments that contained exons were identified by their hybridization with cDNA clones or synthesized oligonucleotide sequences based on cDNA sequence. These restriction fragments were subcloned in the plasmid vector Bluescribe (Stratagene, San Diego, CA). A total of 24 distinct plasmids were subcloned in this manner.

The number and size of each exon was determined by iterations of the following procedure. First, an oligonucleotide-was synthesized that corresponded to the first 20 nucleotides of the cDNA sequence. Using this oligonucleotide as a primer, the plasmid with a genomic insert containing the most 5' exon was sequenced. The resultant sequence was aligned with the cDNA sequence to determine the length of the first exon; the point at which the plasmid and cDNA sequences diverged marked the beginning of the first intron. This exon and the flanking regions were further sequenced using synthetic oligonucleotide primers to generate a continuous nucleotide sequence composed of 5' promoter sequence, exon 1, and the beginning of intron 1. The second and subsequent exons were defined by synthesizing sequencing primers corresponding to the next 20 nucleotides of cDNA sequence that had not been previously assigned to an exon. All exons and the immediately adjacent flanking intron sequences were sequenced in both sense and antisense directions.

The dideoxynucleotide chain termination method of sequencing was carried out using the enzyme Sequenase (United States Biochemical Corporation, Cleveland, Ohio) according to protocols supplied by the manufacturer. The intron region downstream of exon 20 could not be sequenced by this method, due to an unusually problematic repeated sequence in this region that caused a series of 45 stops (bands appeared in all four lanes of the sequencing gel). To resolve this region, sequencing reactions were carried out with Tag polymerase (Perkin-Elmer/Cetus). This enzyme allowed for the polymerization to be performed at 68°C and resolved the bases in this region.

All sequence data were analyzed and screened for overlapping regions using the sequence analysis program Microgenie Sequence Software (Beckman, Palo Alto, CA).

The position of each exon within the restriction map of the gene was determined by hybridization of cDNA fragments or synthetic oligomer sequences to recombinant bacteriophage DNA that had been digested with various restriction endonucleases. The precise location of most exons was subsequently deduced when recognition sequences of endonucleases were identified within the intron-exon sequence and correlated with the map. The position of each of the remaining exons was arbitrarily placed in the center of the smallest restriction fragment to which it hybridized.

The organization of the 27 exons along the genomic map of the retinoblastoma gene is illustrated in figure 4. This figure details the recognition sites for 6 restriction endonucleases and the position of the exons relative to these sites. Exons 1, 2, 3, 6, 9, 10, 13, 21, 22, 23, 24, 235, 26, and 27 have been precisely localized on this map. The other exons were mapped within small restriction fragments and are illustrated in the middle of these fragments. Exons 11, 17, and the cluster of exons 14-16 were mapped by this technique with uncertainties of not more than 2.0 kb. The remaining exons (exons 4, 5, 7, 8, 12, 18, 19, and 20) were all mapped to within 0.8 kb of their true locations. For reference, this map also shows the position of several naturally occurring restriction fragment length polymorphisms.

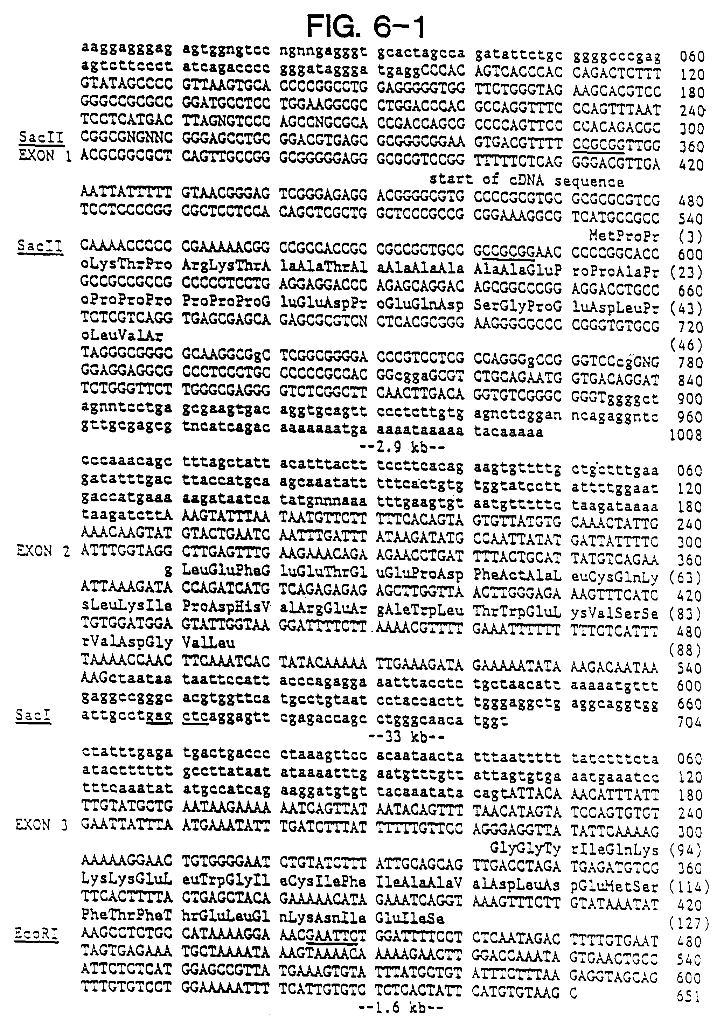

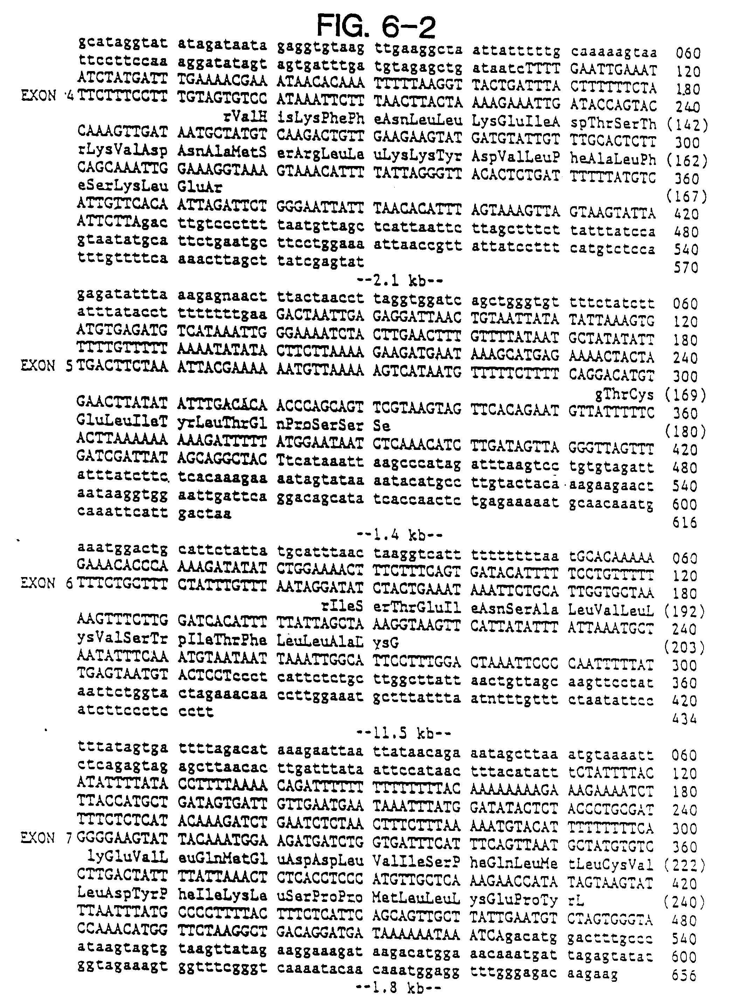

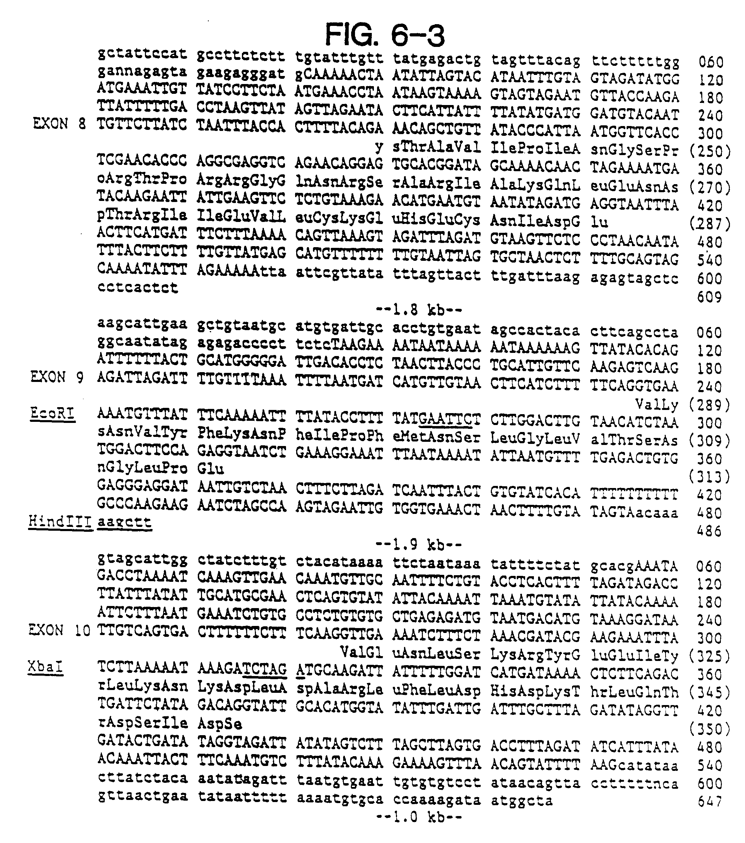

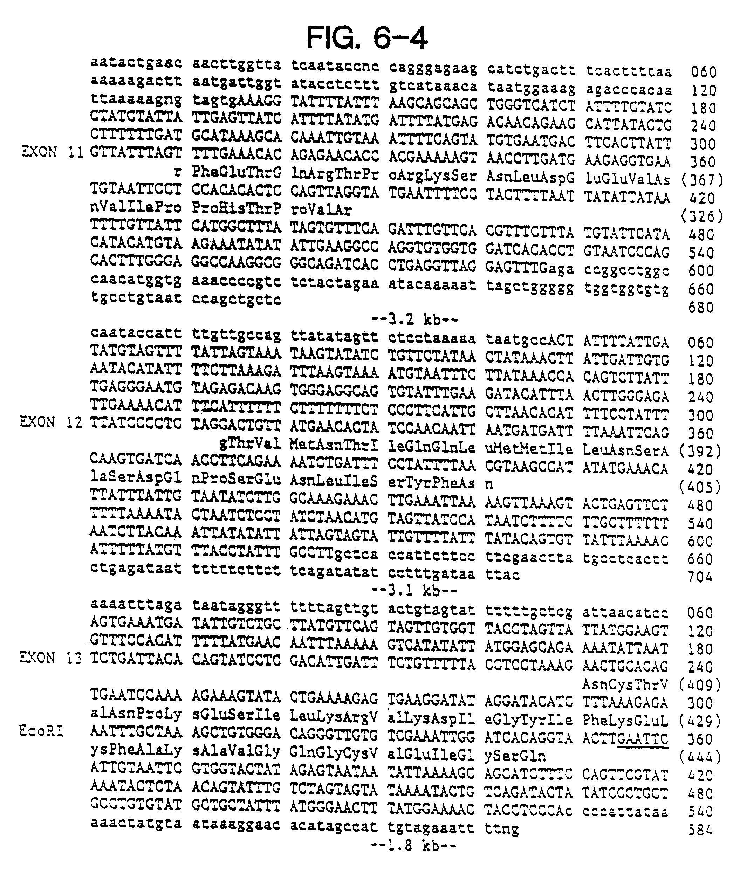

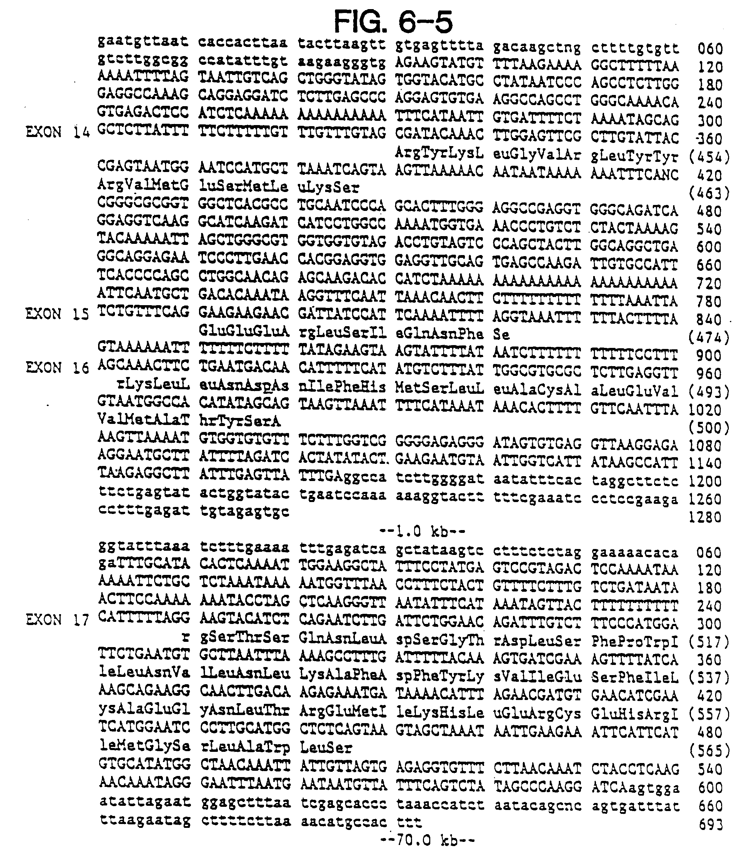

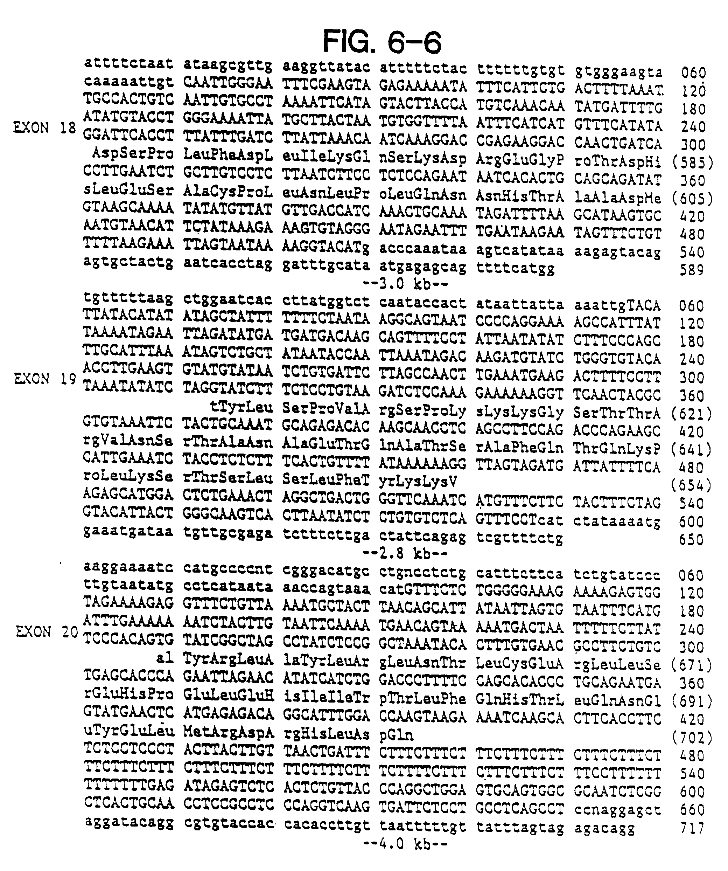

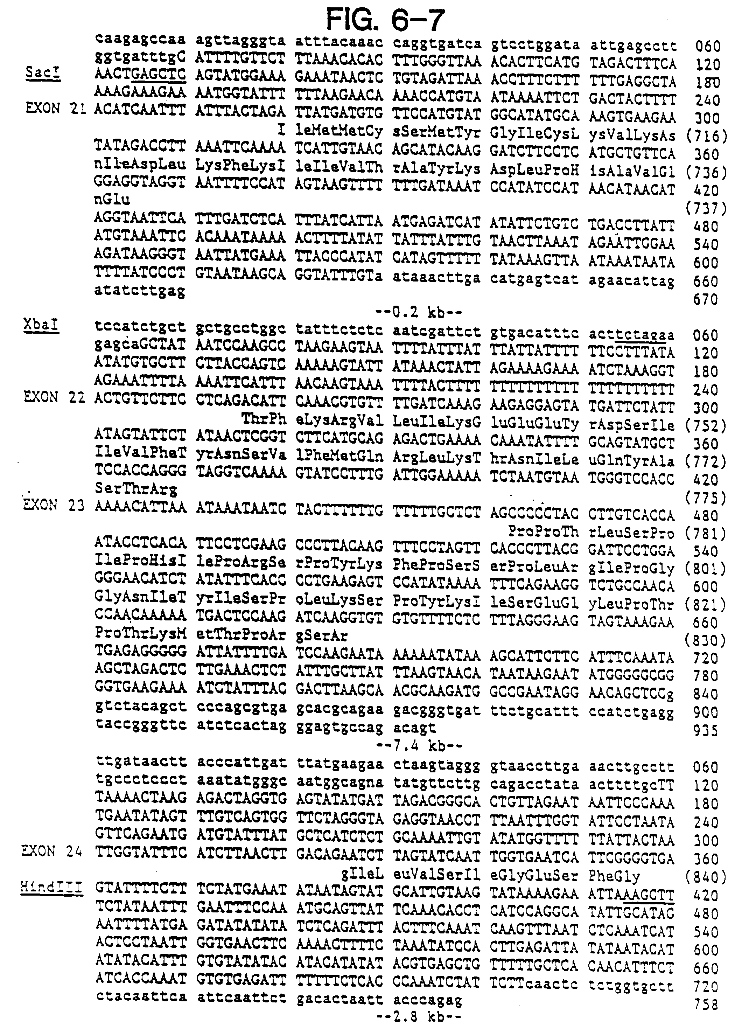

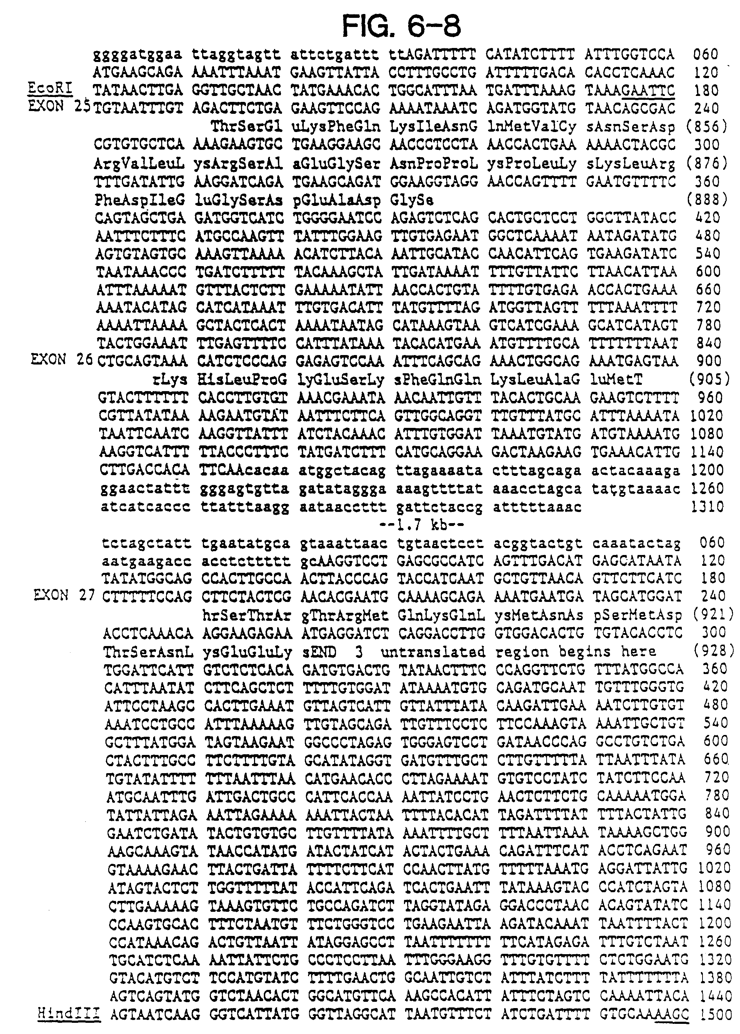

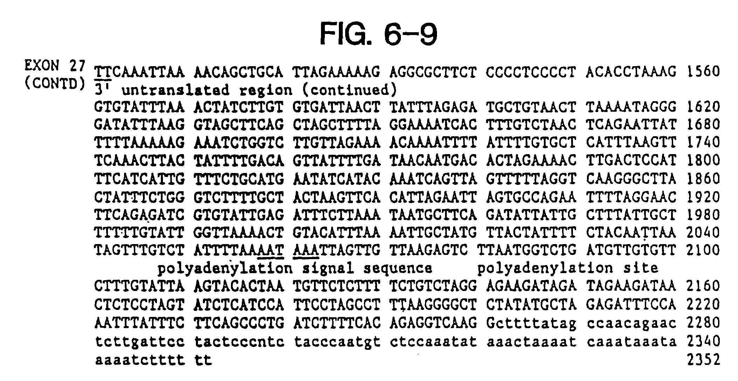

Fig. 6 (6-1 through 6-9) shows the sequence flanking and including each exon. The exons range in size from 31 nucleotides (exon 24), to 1973 nucleotides (exon 27). The shortest intron sequence was found to be 80 nucleotides long and is located between exons 15 and 16, whereas the largest spans approximately 70.5 kb between exon 17 and 18. All of the intron donor and acceptor splicing sites comply with the GT-AG splice junction rule. Our methods of sequencing proved more accurate than previous reports in defining the exact number of exons comprising the retinoblastoma gene.

The first exon and the region immediately 5' to this exon are very G-C rich, which is a characteristic of promoter regions. This region contains 9 possible Hpa II restriction sites, is composed of 66% C+G nucleotides and does not exhibit CpG suppression. These criteria are indicative of a HTF island. This promoter region contained some nucleotides that could not be resolved in either the sense or antisense direction using either Sequenase or Taq polymerase.

Presumably, this was due to secondary structure that forms in this promoter region.

Analysis of the sequence approximately 30 nucleotides upstream of the transcription initiation site defined by Lee et al. (1987b) does not reveal a TATA box that is found in other promoter regions. This suggests that either the previously published initiation site is not in fact correctly defined, or that the retinoblastoma gene lacks the prototypical TATA and CAAT boxes of promoter regions. Further analysis of the sequence 5' to exon 1 reveals a possible TATA box at base pair #-274, labeled base pair #122 in figure 6-1. Homology for the seven base region is only 57%, yet the first four bases T-A-T-A, which are the bases most frequently conserved, are 100% homologous. A possible capping site, CAC, is located 14 bases away and again 49 bases away. CAAT boxes were not identified although the region is generally G-C rich.

The intron sequence that flanks the 3' side of exon 20 consisted of 21 consecutive repeats of the sequence TTT(T)C that together span 87 nucleotides. The number of repeat units can vary between different individuals and the alleles determined by the number of repeats behave like a heritable DNA polymorphism.

A computer search of the sequence data identified several intron regions homologous to Alu repetitive sequences. Alu repeats were located in the following regions: (1) downsteam of exon 2, between bp 492 and bp 704 according to the numbering scheme in Figure 6; 2) upstream of exon 9, between bp 19 and bp 117; 3) downstream of exon 11, between bp 504 and bp 680; 4) the intron sequnce flanking both sides of exon 14 between bp 132 and bp 270 and between bp 420 and bp 741; and, 5) upstream of exon 17, between bp 36 to bp 210. The Alu sequence located downstream of exon 2 contains two internal sequences that are highly conserved in Alu repetitive sequences. The first is a sequence (GAGGCNGAGC) corresponding to the T-antigen binding sequence of the SV40 replication origin. The second is a symmetrical sequence (CCAGCCTGG) of no known function. This short symmetrical sequence is also present in both of the Alu sequences on either side of exon 14. Exons 14 and 15 are separated by a short intron that is almost entirely composed of Alu sequence, suggesting that exons 14 and 15 were possibly at one time a single exon and were divided by the insertion of an Alu element during evolution. This Alu sequence may have been directed to this position by the other Alu sequence located on the 5' end of exon 14 because retroposons have a tendency to integrate adjacaent to one another.

The 3' end of the retinoblastoma gene contains the usual polyadenylation signal sequence, AATAAA. One sequence (TGTGTTCT) located 32 bases downstream of this hexamer is equivalent to the conserved downstream consensus sequence (YGTGTYY) described by McLauchlan et al. (1985). This sequence and surrounding bases compose the "G/T cluster" generally found in a region 30 nucleotides downstream of the polyadenylation signal sequence (Birnstiel et al., 1985).

The p4.7R probe also was used to screen genomic DNA isolated from the tumors of 50 unrelated individuals (40 retinoblastomas, 8 osteosarcomas, and 2 undifferentiated tumors of unknown cellular origin arising in patients with hereditary retinoblastoma), as described in more detail below. These DNA samples were digested with HindIII and analyzed by Southern blot hybridization using radiolabeled p4.7R as the probe. This analysis revealed three types of deviant patterns of the genomic DNA restriction fragments: totally absent fragments, representing apparent homozygous deletions; under-represented fragments, representing apparent heterozygous deletions; and fragments of altered size, reflecting either partial deletion or an alteration of a restriction site. At least 30% of the tumor DNA's exhibited one of these abnormalities. In comparison, Southern blot analysis of leucocyte DNA from 18 normal individuals showed a uniform pattern of restriction fragments.

### Use

The cDNA and genomic sequences, e.g., those in p4.7R, can be used, according to the invention, to screen individuals for the presence of a mutated allele of the Rb gene. This screening procedure will allow individuals having a risk of developing retinoblastoma--because of family history or a previous incidence of retinoblastoma in one eye--to determine the need for routine testing by the current ocular examination procedure. Only if the screening procedure determines that the individual possesses a mutant Rb allele will the examination procedure need to be conducted on a regular basis. Those with two normal Rb alleles can discontinue examination, as the risk of developing retinoblastoma in an individual with two normal copies of the Rb gene is approximately 1 in 20,000, or 0.005%, compared to a risk of 80%-90% if an individual has an Rb allele containing a mutation sufficient to inactivate the allele. Thus, a substantial percentage of individuals who are currently examined regularly are not actually at a greater risk than the general population: neither a family history of nor a previous incidence of retinoblastoma is conclusive evidence that an individual has the genetic predisposition to the disease. Therefore, such individuals, actually carrying two normal copies of the Rb gene, have been repeatedly undergoing the expensive and traumatic ocular examination procedure needlessly.

The screening procedure according to the invention includes: (1) testing a nucleic acid sample of a patient for large deletions in the Rb gene locus; (2) testing a nucleic acid sample of a patient for small deletions or point mutations in the Rb gene locus; and (3) testing a nucleic acid sample of a patient for RFLPs linked to the Rb gene locus.

### Detection of Large Deletions in the Rb Gene

The availability of DNA probes from the Rb gene provides a means of directly detecting genetic lesions that create retinoblastoma-predisposing alleles. Suitable probes include the entire normal retinoblastoma gene sequence, or fragments thereof consisting of 15 or more bases encoding a specific portion of the retinoblastoma gene. When performed by Southern blot and dot blot procedures, this analysis is generally limited to the study of those lesions that create gross structural changes in the Rb gene, such as deletion of many hundreds of base pairs.

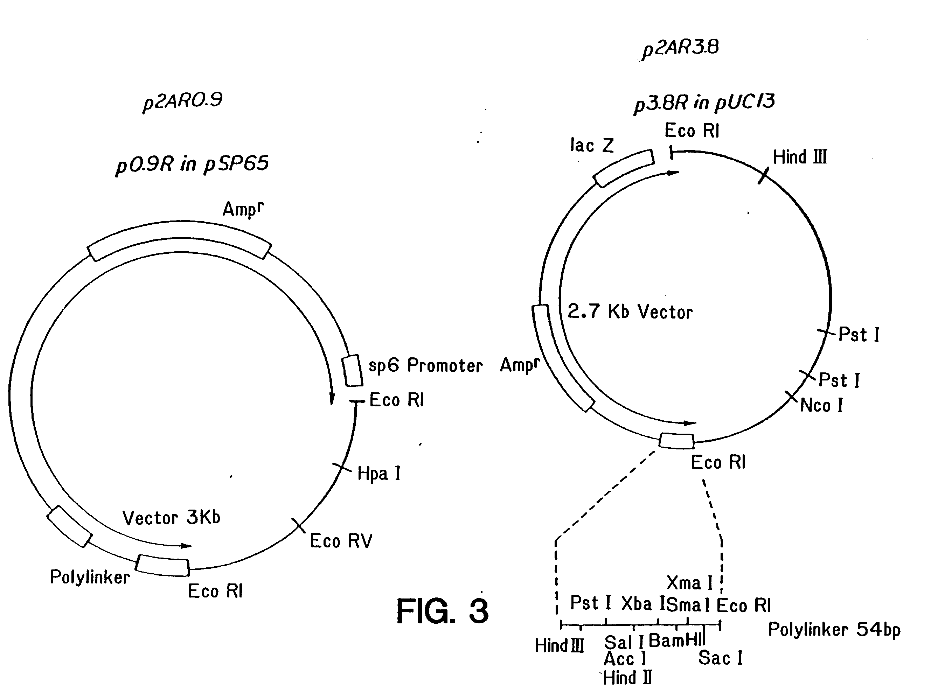

The DNA for a Southern Blot or dot blot analysis is isolated from peripheral leucocytes or, if the patient has had a tumor in one eye, from the tumor. To examine leucocyte DNA, a 10 ml blood sample is obtained from the individual, and the genomic DNA is isolated from the leucocytes in the sample, according to standard techniques. This DNA is digested with a restriction endonuclease (e.g., HindIII), and the resulting fragments are separated on an agarose electrophoresis gel according to a physical property such as molecular shape or molecular weight. For the purposes of this invention, molecular shape is defined as the structural configuration of the molecule (e.g., linear, circular, double-stranded or single-stranded). The DNA in the gel is transferred to a nitrocellulose filter by blotting. The filter is then probed with, e.g., radiolabeled p2AR3.8 and, separately, p2AR0.9, containing subfragments from p4.7R obtained by EcoRl digestion. (The diagrams of the vectors p2AR3.8 and p2AR0.9 are shown in Figure 3.) In order to more precisely define the location of any abnormalities detected, two or more subfragment probes are used separately rather than the entire p4.7R insert probe. The autoradiograms of the probed filter generate the data necessary to construct a restriction map of the Rb locus in the somatic or tumor DNA of the tested individual.

This restriction map is compared with a control restriction map, determined by using the same restriction enzymes for digestion and the same probe. A suitable control is DNA obtained from an adenovirus-transformed retinal cell line or leucocyte DNA from a set of normal individuals. If the tested individual has an Rb allele containing a significantly large deletion, a restriction map of his DNA, compared with the control, will contain an additional band or bands, and/or a band or bands that have lost 50% of their intensity, caused by a change in the size, or total elimination, of one or more restriction fragments by the deletion in one allele at the Rb locus.

This screening procedure by Southern analysis will detect the existence of Rb alleles which have large deletions and are thereby non-functional. If this analysis indicates that the tested DNA from an individual has a restriction map which is different from the control map, there is a high probability that the individual contains a non-functional, mutant Rb allele. The individual must be monitored closely thereafter for the development of retinoblastoma.

If the test restriction map appears identical to the control, a different screening procedure can be performed to determine if the individual possesses an Rb allele having a small deletion or point mutation. Small deletions and point mutations may be sufficient to inactivate the allele, but not prevent hybridization with a probe. An example of this screening procedure is outlined below.

### Detection of Other Mutations in the Rb Gene

To examine a DNA sample of an individual for small deletions or point mutations in the Rb locus, both homologs of the Rb gene from said individual are cloned. The cloned alleles then can be tested for the presence of nucleic acid sequence differences from the normal allele, e.g., as represented by p4.7R, by one of the following two methods: (1) the nucleotide sequence of both the cloned alleles and p4.7R are determined and then compared, or (2) the RNA transcripts from p4.7R are hybridized to single stranded whole genomic DNA from an individual to be tested, and the resulting heteroduplex is treated with Ribonuclease A (RNase A) and run on a denaturing gel to detect the location of any mismatches. In more detail, these methods can be carried out according to the following procedure.

The alleles of the Rb gene in an individual to be tested are cloned using conventional techniques. A common method, for example, employs the bacteriophage vector EMBL3 (Frischauf et al., 1983, J. Mol. Biol., Vol. 170, pp. 827-). A 10 ml blood sample is obtained from the individual. The genomic DNA isolated from the cells in this sample is partially digested with MboI to an average fragment size of approximately 20 kb. Fragments in the range from 18-21 kb are isolated. The resulting MboI-ended fragments are ligated into the EMBL3 vector DNA which has been completely digested with BamHI, treated with alkaline phosphatase, and heated to 68°C for 10 minutes to disrupt the cohesive ends. This ligation mix is used in an in vitro lambda packaging reaction, and the packaged phage are amplified by growing a plate stock. (This cloning technique is described generally in Maniatis et al., 1982, Molecular Cloning: A Laboratory Manual, Cold Spring Harbor Publications, pp. 256-293.)

Approximately 5 x 10⁵ plaque forming units (pfu) from this plate stock are then screened with radiolabeled p4.7R by hybridization and autoradiography. Plaques which show hybridization to the p4.7R probe are plaque-purified and rescreened according to the above procedure. Positive plaques from the rescreening are isolated and used to prepare DNA putatively containing Rb alleles from the individual.

The MboI genomic inserts in these isolated EMBL3 vector DNA samples are tested for the location of the sequences homologous to p4.7R by Southern analysis. DNA samples containing the entire Rb gene region are selected, and the appropriate restriction fragments containing the Rb gene from these samples are subcloned into a suitable vector, such as pUC9. These subclones thus contain copies of one or both Rb alleles from the DNA of the individual to be tested. To determine if both alleles are represented, the initial phage isolates are tested for the existence of restriction polymorphisms. These subcloned alleles are then examined for differences from p4.7R by one of the following techniques.

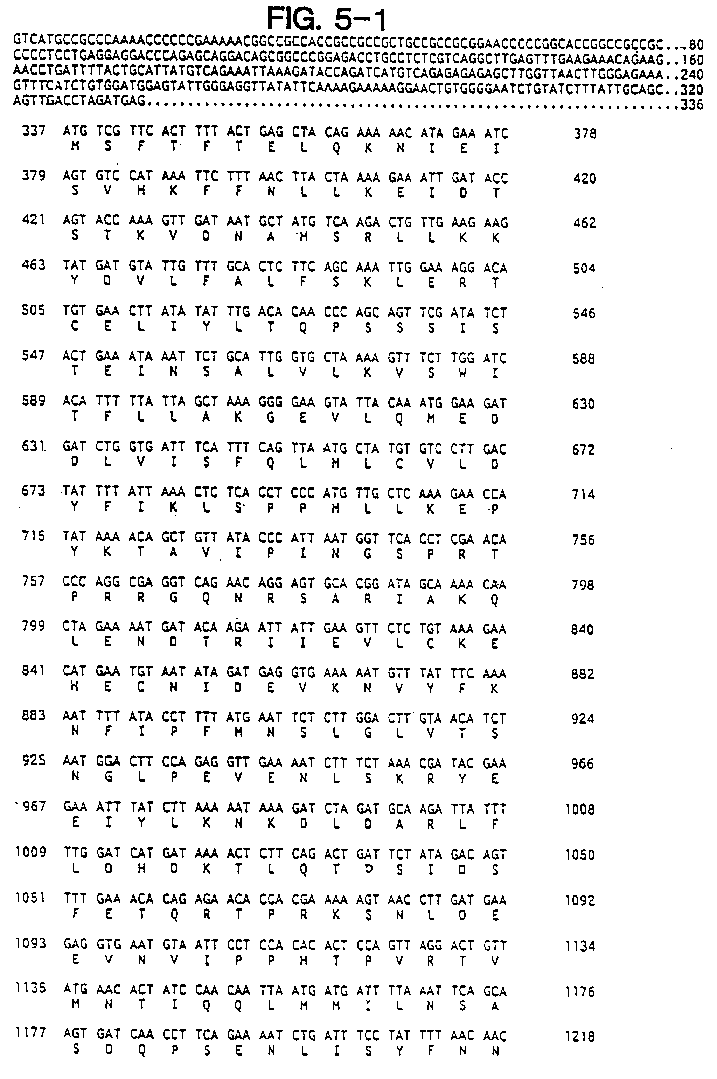

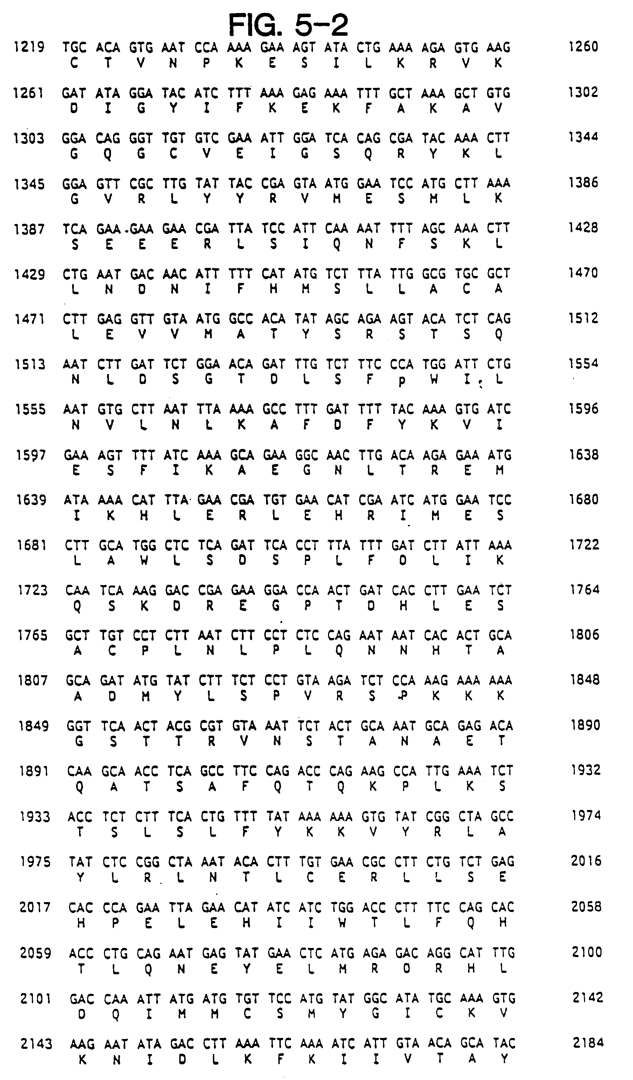

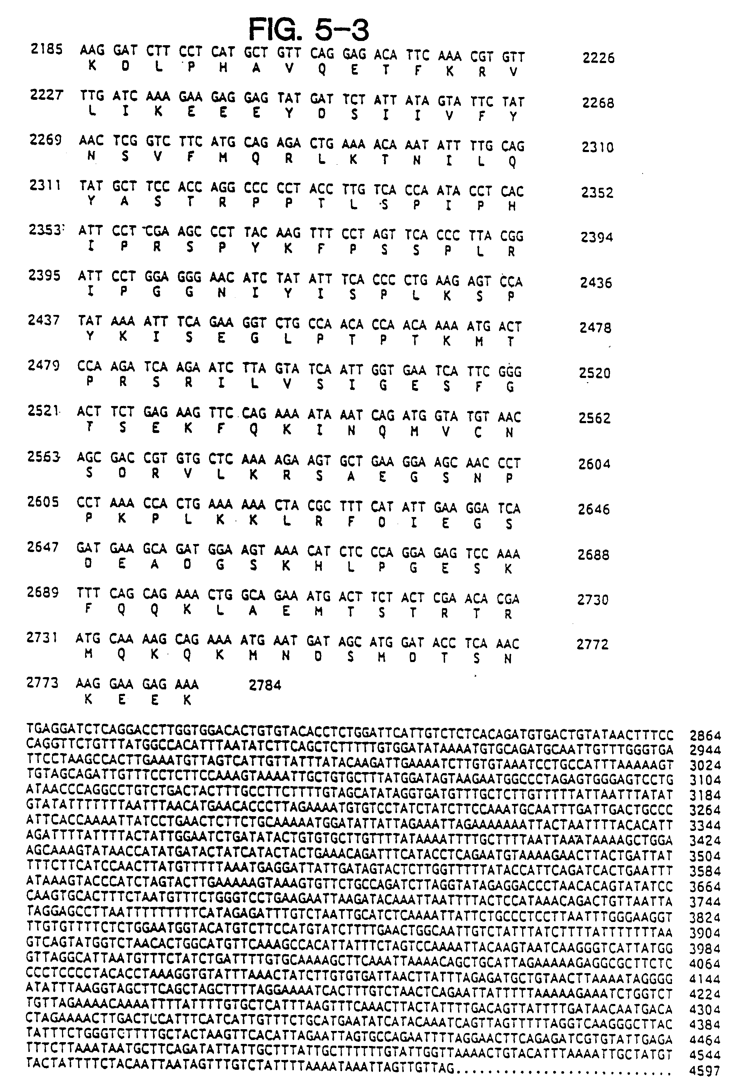

First, the nucleotide sequence of the normal Rb gene in p4.7R is determined. Restriction fragments of approximately 500 base pairs (bp) from p4.7R are subcloned into an M13mp8 phage vector and sequenced by the dideoxy technique (Sanger et al., 1977, Proc. Natl. Acad. Sci. USA, Vol. 74, pp. 5463-). A composite sequence of the Rb gene then can be assembled from these individual subclone sequences. The complete sequence of the normal retinoblastoma gene and flanking sequences is shown in Fig. 5 and Fig. 6.

The isolated Rb gene alleles are sequenced according to the following procedure. Restriction fragments (- 2kb) of the allele are subcloned into the M13mp8 vector, and short stretches (∼500 bp) are sequenced individually using small restriction fragments isolated from p4.7R as the primers in the dideoxy sequencing reactions. The composite nucleotide sequence of the isolated allele then can be constructed from these individually-primed sequences. This sequence is compared directly with the sequence of the normal Rb gene, determined from p4.7R, to reveal any deletions or point mutations in the isolated allele.

An alternative method of comparing the allelic DNA with the normal Rb gene employs RNase A to assist in the detection of differences between the p4.7R sequence and the allele sequence. This comparison is performed in steps using small (∼500 bp) restriction fragments of p4.7R as the probe. First, p4.7R is digested with a restriction enzyme(s) that cuts the Rb gene sequence into fragments of approximately 500bp. These fragements are separated on an electrophoresis gel, purified from the gel and cloned individually, in both orientations, into an SP6 vector (e.g., pSP64 or pSP65; Melton et al., 1984, Nucleic Acids Res., Vol. 12, pp. 7035-). The SP6-based plasmids containing inserts of p4.7R fragments are transcribed in vitro using the SP6 transcription system, well known in the art, in the presence of [α-³²P]GTP, generating radiolabeled RNA transcripts of both strands of the cDNA of the Rb gene.

Individually, these RNA transcripts are used to form heteroduplexes with the allelic DNA, as described by Myers et al., 1985, Science, Vol. 230, pp. 1242-46, the teachings of which are incorporated herein by reference. Mismatches that occur in the RNA:DNA heteroduplex, owing to sequence differences between the p4.7R fragment and the Rb allele subclone from the individual, result in cleavage in the RNA. strand when treated with RNase A. Such mismatches can be the result of point mutations or small deletions in the individual's Rb allele. Cleavage of the RNA strand yields two or more small RNA fragments, which run faster on the denaturing gel than the RNA probe itself.

In the RNAse A technique, radiolabeled Rb gene RNA is hybridized to single strands of an individual's Rb alleles which have been cloned into a vector. The RNase A technique is advantageous, however, because it also can be used without having to clone the Rb alleles. Preferably, genomic DNA is isolated from blood cells of the individual to be tested, and this genomic DNA is hybridized directly with the radiolabeled Rb RNA probes to determine sequence differences from the normal Rb gene. Specifically, 5 µg of isolated, total genomic DNA is resuspended with the labeled RNA probe in 30 µl of hybridization buffer (80% formamide, 40mM Pipes pH6.4, 0.4M NaCl, and 1mM EDTA), and this hybridization mix is treated at 90°C for 10 minutes to denature the DNA. The mixture then is cooled slowly to 45°C and incubated at this temperature for 10 hours to allow hybridization of the RNA probe to the single-stranded DNA copies of the Rb allele. After hybridization, RNase A treatment and electrophoresis are performed as described by Myers et al., supra. Mismatches between the RNA probe and the genomic copies of the individual's Rb alleles are then readily detected.

### Detection of RFLPs Linked to the Rb Gene

The inheritance of a retinoblastoma- predisposing defect can be traced by following its co-inheritance with DNA polymorphisms in a pedigree analysis.

The gene map shown in Figure 2 was used to develop nucleic acid probes useful for retinoblastoma diagnosis. To do so, the bacteriophage DNA corresponding to the human inserts were subcloned in the plasmid vector "Bluescribe" (Stratagene). Fifteen single-copy DNA fragments from the gene, ranging in size from 500 bp to 2000 bp, were subcloned. These sequences are scattered over the 200 kb of the mapped region. Subcloned DNA fragments were separated from vector sequences by digestion of plasmid DNA with one or more restriction endonucleases, electrophoresis through a 0.6% low-melting-point agarose gel, and purification by chromatography using an Elutip-d column (Schleicher and Schuell). Purified DNA fragments were radiolabeled with ³²P-dCTP (New England Nuclear) by the random primer technique using the Klenow fragment of DNA polymerase I.

Restriction fragment length polymorphisms (RFLP's) were discovered by digesting genomic DNA isolated from six normal individuals with 33 different restriction enzymes. The DNA fragments resulting from the 198 separate digests were separated on a 0.8% agarose electrophoresis gel according to molecular shape or molecular weight. The DNA was transferred to nitrocellulose filters and hybridized with single copy DNA probes purified from the retinoblastoma gene according to published methods (T.P. Dryja et al., 1986, Proc. Natl. Acad. Sci. USA, Vol. 83, pp. 7391-94).

Of the 15 single-copy probe fragments, only five reveal RFLP's. Four of the polymorphisms appear to be the result of minor alterations (perhaps single base changes) in the recognition sequence of a restriction endonuclease (KpnI, XbaI, MboII, or Tth111I). The fifth polymorphism reflects variability in the number of tandem repeats of a 50 base pair sequence. The location of the DNA polymorphisms are shown in the map in Figure 2 (vertical arrows above the map). The location of the polymorphic MboII site(s) has not been determined precisely but is located at approximately 175 kb on this map. The frequencies of alleles which correspond to particular DNA polymorphisms are indicated in Table 1.

In order to demonstrate the utility of these probes to detect the presence of retinoblastoma-predisposing alleles in humans, twenty pedigrees with hereditary retinoblastoma were analyzed. DNA was extracted from leucocyte nuclei of venous blood from available family members according to the method of Kunkel et al., 1977, Proc. Natl. Acad. Sci. USA, Vol. 74, pp. 1245-49, hereby incorporated by reference. For analysis of a kindred with a given RFLP, DNA from the available family members was digested with the appropriate restriction endonuclease. The resulting fragments were separated by agarose-gel electrophoresis, transferred to nitrocellulose filters, and hybridized to labeled probe.

In these families, the inheritance of alleles determined by the DNA polymorphisms within the retinoblastoma gene were traced and compared to the inheritance of the retinoblastoma-predisposing trait. For example, consider the polymorphism detected by probe p68RS2.0 (see Table 1). When genomic DNA is digested with the restriction enzyme RsaI, this probe hybridizes to allelic DNA fragments of different lengths. The size of these fragments ranges from 1.5 kb to 2.0 kb with intervals of approximately 50 bp. The DNA sequence of the 2.0 kb genomic fragment cloned in p68RS2.0 has a 50 to 53 bp segment which is repeated approximately 30 times (Table 2). This 53bp segment can be used as a probe in these analyses. A portion of the repeated sequence has homology to core sequences of VNTR's (Variable Number of Tandem Repeat) reported elsewhere (Y. Nakamura et al., 1987, Science, Vol. 235, pp. 1616-22). (The 11 bp sequence shown in Table 2 above the repeat unit represents the core sequence reported for some VNTR's observed by Nakamura et al.) Because such tandemly repeated sequences tend to be genetically unstable, the number of repeats is highly variable. Eight distinct alleles at this site have been detected, and more may exist. Because of this number of common alleles. seventy-five percent of unrelated individuals are heterozygous for this polymorphism. The high frequency of heterozygosity makes this polymorphism extremely useful. (In Table 2, the brackets "(" and ")" denote regions of variability within the repeat unit; and the bases underlined above and below the bracketed regions denote possible alternate bases for those regions of variability.)

**TABLE 1**

| DNA POLYMORPHISMS IDENTIFIED WITHIN THE RETINOBLASTOMA GENE | | | | |
|---|---|---|---|---|
| DNA PROBE | RESTRICTION ENDONUCLEASE | MAP LOCATION¹ | ALLELES | |
| | | | SIZE (Kb)³ | FREQUENCY⁴ |
| p68RS2.0 | RsaI | 142-143 kb | 2.00 | 0.13 |
| | | | 1.95 | 0.02 |
| | | | 1.90 | 0.07 |
| | | | 1.85 | 0.07 |
| | | | 1.80 | 0.35 |
| | | | 1.75 | 0.20 |
| | | | 1.65 | 0.09 |
| | | | 1.50 | 0.09 |
| | | | | |
| p88PR0.6⁵ | XbaI | 120 kb | 7.0 | 0.55 |
| | | | 5.5 | 0.45 |
| | | | | |
| p35R0.6 | Tth111I | 195 kb | 4.95 | 0.20 |
| | | | 4.35 | 0.80 |
| | | | | |
| p2P0.3 | MboII | 175 kb² | 1.0 | >.90 |
| | | | 0.8 | <.05 |
| | | | 0.6 | <.05 |
| | | | 0.3 | <.05 |
| | | | | |
| p95HS0.5 | KpnI | 25 kb | 12.0 | 0.95 |
| | | | 8.0 | 0.05 |

| | | | | |
|---|---|---|---|---|
| ¹The map location of each polymorphic site refers to the position on the restriction map of the gene shown in Figure 1. | | | | |
| ²The location of the MboII site is approximate, since the precise position of this site within the map is not yet known. | | | | |
| ³The allele sizes were calculated from several indepedent measurements using HindIII fragments of lambda phage DNA as a standard. | | | | |
| ⁴Allele frequencies are based on a population of 40-60 unrelated individuals. | | | | |
| ⁵p88PRO.6, p35RO.6, p2PO.3 and p95HS0.5 are probes isolated from other regions of the retinoblastoma gene, shown in Fig. 2. | | | | |

represents the core sequence reported for some VNTR's observed by Nakamura et al.) Because such tandemly repeated sequences tend to be genetically unstable, the number of repeats is highly variable. Eight distinct alleles at this site have been detected, and more may exist. Because of this number of common alleles, seventy-five percent of unrelated individuals are heterozygous for this polymorphism. The high frequency of heterozygosity makes this polymorphism extremely useful. (In Table 2, the brackets "(" and ")" denote regions of variability within the repeat unit; and the bases underlined above and below the bracketed regions denote possible alternate bases for those regions of variability.)

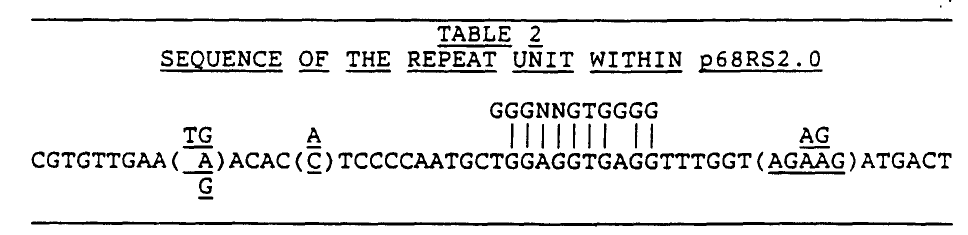

Fourteen retinoblastoma families carried constellations of alleles at this DNA polymorphism. This variation allows an examination of the frequency of co-inheritance of this site with the retinoblastoma-predisposing trait. For example, genomic DNA of individuals in these families was digested with Rsa I and the co-inheritance of any one Rsa I fragment with defective Rb alleles determined.

### Sequence Analysis

RFLP analysis can reveal only those sequence variations that give rise to a restriction fragment that is detectably different on a Southern blot from restriction fragments characterizing the normal nucleic acid. Most such detectable polymorphisms result from DNA sequence variation within a restriction endonuclease recognition site. These sites are rare, and finding them requires a laborious and expensive screening process in which genomic DNAs from several unrelated individuals are digested with as many as 50 different restriction enzymes. The fraction of all genomic DNA sequence polymorphisms (DSPs) at a specific locus that can be detected as RFLPs is small and depends on the number of enzymes used for screening; generally 90% or more of the DNA sequence polymorphism in the human genome is not within reach of RFLP-based analysis. Tens or even hundreds of potentially useful DSPs may exist within or near most disease-causing genes, but often only a few and sometimes none of these DSPs are detectabale as RFLPs. Fig. 4 shows a scale map of the 200 kilobase genomic region that includes the 27 exons of the human retinoblastoma gene. The 27 exons make up a 4.7 kilobase transcript.

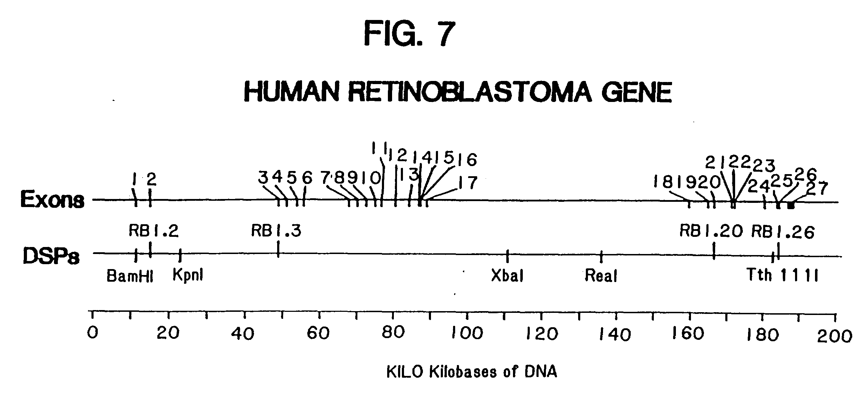

Fig. 7 shows the locations of the DNA sequence polymorphisms (DSPs) identified in this gene. Polymorphisms identified by the name of a restriction enzyme are RFLPs; polymorphisms RB1.2, RB1.3, RB1.20 and RB1.26 are not detectable as RFLPs, and were found by PCR-amplification and direct sequencing, as described below. The 200 kilobase genomic region was isolated in a series of overlapping inserts from 35 distinct recombinant bacteriophage lambda clones. Exon-containing segments were subcloned into bluescribe plasmid cloning vectors (Stratagene, Inc.). Initial sequencing of cloned plasmid inserts was carried out using conventional methods for plasmid sequencing. Based on this genomic sequence, pairs of 20-base oligonucleotide primers were synthesized so that numerous regions 320 - 1200 bp in size could be amplified from genomic DNA by the polymerase chain reaction (PCR) method of Mullis et al.

For each amplification reaction, from 200 ng to 1.0 ug of genomic DNA was prepared in a reaction buffer containing 20 mM Tris (pH 8.4 or pH 8.6), 30 ug/ml bovine serum albumin, 300 mM to 7.5 mM, 10-50 pM of each oligonucleotide primer, and 1 unit of Taq polymerase (Perkin-Elmer Cetus). Optimal MgCl₂ concentrations and pH of the PCR reactions varied depending on the primer pair. PCR-amplification (30-35 rounds) was carried out following a cycle of 10 seconds at 94°C (denaturation), 10 seconds at 42-55°C (annealing), and 30 seconds at 70°C (Polymerization) using a programmable thermal cycler (Ericomp Corp., San Diego). All times are based on sample temperature rather than heat-block temperature, and do not include 'ramping time' for the heat block. Optimal annealing temperatures varied for each primer pair. This protocol allowed PCR-amplification of regions as large as 2500 bp. Genomic DNA from 9-20 individuals was amplified for each region and screened for DSP.

In order to detect and utilize a greater fraction of the existing DNA sequence polymorphism in the retinoblastoma gene, we applied techniques of polymerase chain reaction (PCR), generally as described by K.B. Mullis et al., 1987, Methods Enzymol., Vol. 155, pp. 335-51, and direct sequencing, generally as described by C. Wong et al., 1987, Nature, Vol. 330, 384-86, to analyse normal allelic variation at this locus. Oligonucleotide primers were synthesized to amplify regions from the gene that varied in size from 320 - 1200 bp. Amplification and sequencing were carried out on DNA from at least 9 unrelated individuals for all regions screened, though for many regions 15 or more individuals were analyzed. In most cases, primer pairs were derived from intronic sequences that flanked one of the 27 exons of the gene, such that the PCR-amplified region contained both intron and exon sequences.

The results of this screening process are shown in Tables 3 and 4. Amplified DNA sequences were compared to one another and checked against sequence data from previously cloned plasmid inserts derived from the same region. Bases obscured by technical artifacts or other ambiguities were not tabulated. Of 3712 bp of genomic DNA sequence screened at this locus, four sequence variations were identified (Table 3; map locations are shown in Fig. 7). All four variations were found in introns; of these, one is likely a rare variant (found in only 1 or 15 individuals sequenced), and three represent bona fide DNA sequence polymorphisms. A representative example (RB1.3) is illustrated in Fig. 7. This polymorphism occurs near exon 3 of the retinoblastoma gene. Neither form of the polymorphic sequence forms the recognition site of a known restriction enzyme, and hence this DSP is not detectable as an RFLP. Among a total of 82 genetically distinct (from unrelated individuals) alleles examined, no other base was observed at this site.

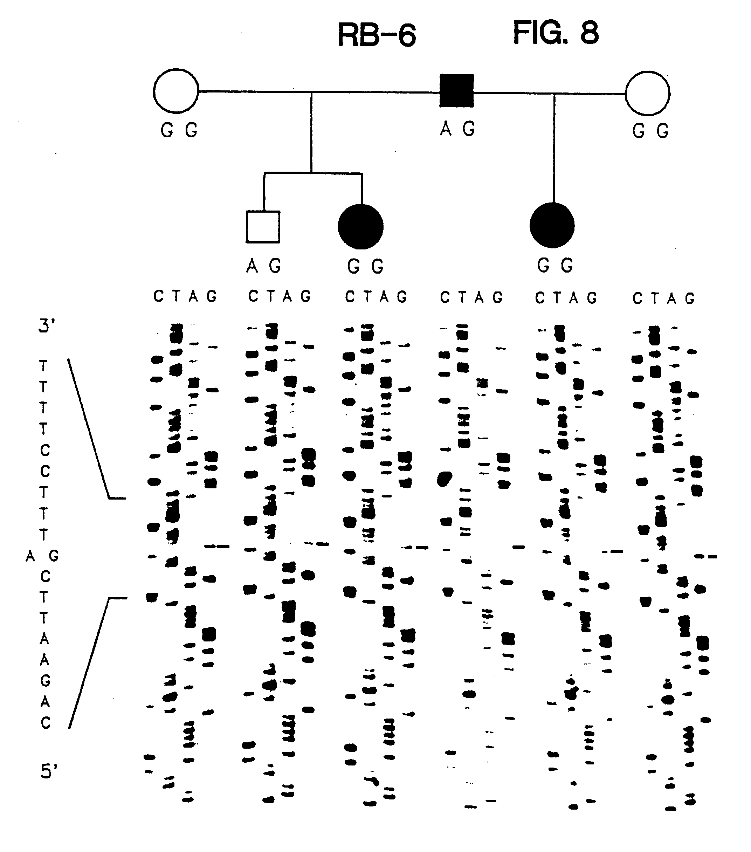

Fig. 8 illustrates the inheritance of the polymorphism RB1.3 in a retinoblastoma-prone family. Oligonucleotide primers (see Table 4) were used to PCR-amplify a 530 bp region of the human retinoblastoma gene that includes exon 3. The amplified fragment was sequenced by the methods described below. The sequence surrounding the polymorphism is written at the left side of the figure, read 5' to 3' from bottom to top, and the polymorphic bases are identified by adjacent tic marks.

The details of the analysis were as follows. Prior to sequencing, all PCR-amplified DNA samples were treated with proteinase-K and extracted with phenol/chloroform. High molecular weight DNA was separated from unused dNTPs and oligonucleotide primers by column purification through sepharose CL-6B (Pharmacia). 250-400 ng of double-stranded PCRF-amplified template was combined with 1-2 pM of (³²P) end-labeled sequencing primer, and heat-denatured for 3 minutes at 96°C. This primer-template mixture was added to a buffer containing: MgCL₂ (2.5 mM), Tris-HCL pH 7.5 (5 mM), 6 units Sequenase (U.S. Biochemical) and dithiothreitol (3 mM), and divided into 4 reaction mixtures each containing all four deoxynucleotides (32 uM each) and one dideoxynucleotide (5 uM). This mixture was immediately incubated for 5 minutes at 37-42°C, and polymerization was stopped with a 0.37% EDTA stop buffer. Prior to loading on sequencing gels, the samples were heat denatured at 96°C for 2 minutes. Conventional 0.4 mm thick, 6% polyacrylamide sequencing gels were used, and autoradiography was typically for 12-24 hours without an intensifying screen.

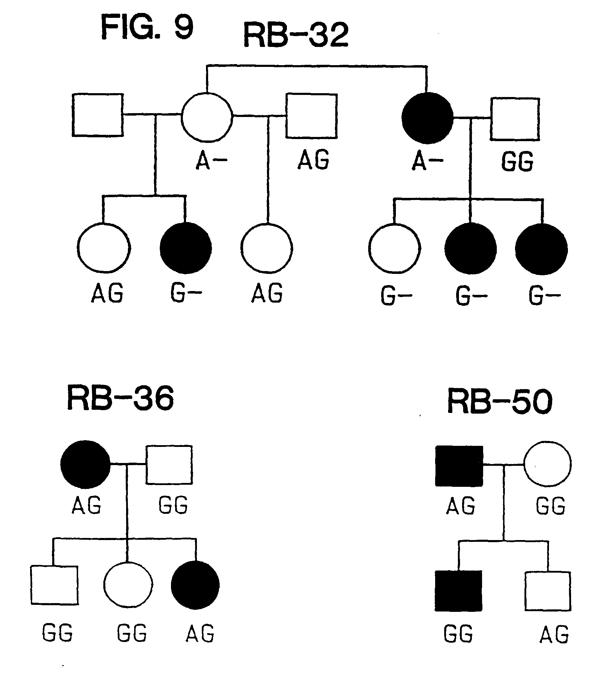

Fig. 9 illustrates segregation of the DSP RB1.3 in three families with hereditary retinoblastoma. Alleles are shown beneath the symbol for each person. Affected individuals are indicated by filled symbols. In family RB-32, the (-) allele is the result of an intragenic deletion. By subsequent Southern blotting studies, the deletion was found to extend from exon 2 to exon 17. Based on these results, it can be predicted that the unaffected members of family RB-32 who carry the (G,-) genotype are also carriers of the mutation.

The DSPs we have detected are valuable genetic markers for our studies of hereditary retinoblastoma. In its hereditary form, a predisposition to the disease is passed from affected individuals to their offspring as a dominant trait with 90% penetrance. It can be seen from Fig. 8 that the affected father, who has passed the disease to two children, is heterozygous for RB1.3. Both affected children received the G allele, while the unaffected child inherited the allele marked by an A at this polymorphic site. In this family, then, inheritance of the G allele from the affected parent is in phase with and diagnostic for the disease-predisposing phenotype. Fig. 9 shows our analysis of three other retinoblastoma-prone families using RB1.3. Inheritance of the polymorphic markers we describe here has followed the expected Mendelian pattern in every family examined so far. No cross-overs were observed between the polymorphic sites and the retinoblastoma-predisposing trait in any of the pedigrees. This follows our expectations since the polymorphisms are within the disease gene. In family RB-32, an intragenic deletion in one copy of the RB gene, presumably causing the predisposition to the tumor, was identified by Southern blotting (data not shown). The deletion includes the region surrounding RB1.3, and hence carriers of the disease-predisposing allele are genotypically hemizygous for the A allele (A,-). Two unaffected members of pedigree RB-32 are carriers for the disease-predisposition, based on analysis of RB1.3 (see Fig. 9). More happily, Fig. 9 shows that the other unaffected children in pedigree RB-32, as well as those in pedigrees RB-36 and RB-50, are not carriers of the cancer-predisposition and therefore will not pass the disease on to their children. These results highlight the diagnostic value of this class of human genetic markers that were heretofore unavailable for this purpose.

The data we present may also be used to estimate the level of heterozyygosity in the human genome from a novel perspective. Previous estimates based on restriction enzyme screening may be subject to a bias because the sequences recognized by these enzymes do not necessarily reflect a random sampling. It is likely that a substantially higher level of polymorphism occurs at CpG pairs than elsewhere. This is reflected by the relatively high proportion of RFLPs revealed by such enzymes as Msp I (CCGG) and Taq I (TCGA). The method we describe is not subject to this bias. From the results of our screening, it can be calculated that genomic heterozygosity at this locus is approximately h=0.00039. If only the intron seqeunces are considered, this estimate increases to h=0.00070. These estimates are below the predictions of others, and may reflect the absence from our methods of the bias described above. However, an analogous calculation of heterozygosity (0.00044 < h < 0.00087) based instead on our initial RFLP screening is also below the estimates of others and is quite consistent with our estimate based on direct sequencing. It seems likely that the human retinoblastoma gene is intrinsically less polymorphic than many other regions of the genome. Although mutations in this gene are known to be early events in the formation of several types of cancer, it is unclear why polymorphism at this locus may have been selected against in human evolution.

The approach for detecting DSPs demonstrated here has several advantages over conventional RFlP-based screening. As we have argued, DSP screening by amplification and direct sequencing could increase by an order of magnitude the number of available polymorphic markers at any cloned locus. This technique encompasses and supercedes restriction enzyme-based screening since RFLPs and VNTRs may also be detected. As the only requirement for utilization of such markers is knowledge of a unique set of amplification primer sequences and of the polymorphism itself, publication of a polymorphism immediately makes it available to all readers. Hence, problems and delays associated with the physical transfer of plasmid DNAs between laboratories are-avoided, and the costs of maintaining plasmid repositories will be ultimately reduced. In addition, rapid analysis of these polymorphic markers can be carried out on a large scale with the use of allele-specific oligonucleotide probes for direct hybridization to amplified DNA. Finally, based on our experience with both strategies at the same locus, we found the expense and effort required to locate DSPs by either method to be comparable.

### Treatment of Patients Having a Defective Rb Gene

In addition to screening, the invention includes polypeptide therapy for those individuals determined to contain a defective Rb allele, and who therefore are at risk of developing retinoblastoma.

To prevent the formation of retinoblastoma in these individuals, the Rb polypeptide is administered therapeutically in an amount sufficient to inhibit retinoblastoma tumor formation or growth (anti-retinoblastoma-forming amount). An anti-retinoblastoma-forming dosage of the Rb polypeptide is 1 to 500 µg/kilogram of body weight/day. The Rb protein can be administered by injection with a pharmacologically acceptable carrier, either alone or in combination with another agent. Acceptable pharmacological carriers are those which dissolve the Rb polypeptide or hold it in suspension, and which are not toxic to the extent of permanently harming the patient. Preferred are aqueous solutions of salts or non-ionic compounds such as sodium chloride or glucose, most preferably at an isotonic concentration. Other agents may be present provided that they do not interfere with the action of the Rb polypeptide. Those skilled in the art will know, or will be able to ascertain with no more than routine experimentation, particular pharmacological carriers for this composition.

Rb polypeptide suitable for therapy can be prepared by any one of the following three conventional procedures. First, the Rb polypeptide can be produced by cloning the Rb cDNA from p4.7R into an appropriate mammalian expression vector, expressing the Rb gene product from this vector in an in vitro expression system, and isolating the Rb polypeptide from the medium or cells of the expression system. General expression vectors and systems are well known in the art.

Second, the Rb polypeptide can be produced using protein chemistry techniques, wherein the specific amino acid residues are joined together synthetically in the appropriate sequence.

Third, naturally occurring Rb protein can be isolated from total protein samples by affinity chromatography. Antibodies specific for the Rb protein are prepared by standard procedures (see below) and coupled to an inert matrix, which is then used to selectively bind the Rb proteins.

### Immunodiagnosis of Retinoblastoma

This invention also includes methods for determining whether a particular tumor is the result of an Rb gene abnormality. Since osteosarcomas and certain undifferentiated tumors can result from detectable lesions in the Rb gene, immunodiagnosis can be used to aid in the diagnosis of such tumors.

In order to produce anti-Rb antibody, a rabbit is immunized with either naturally occurring Rb protein or Rb polypeptide produced as described above. The anti-Rb antibody generated is then labeled, e.g., radioactively, fluorescently, or with an enzyme such as alkaline phosphatase. The labeled antibody is used to determine whether human tumors are of defective Rb gene origin. This can be carried out using any conventional technique. For example, the tumor sample can be liquified and tested against the labeled antibody using a conventional ELISA (Enzyme-linked immunosorbent assay) format. Alternatively, human tissue samples (e.g., biopsy samples) can be tested for expression of the retinoblastoma protein by other immunological techniques, see e.g., I. Roitt, Interaction of Antigen and Antibody, In Essential Immunology, Fifth edition, Boston: Blackwell Scientific Publications, 1984, pp. 145-75.

Immune complexes will be detected in tumor samples which have antigens (e.g., retinoblastoma polypeptide) reactive with anti-Rb antibody. Tumors which lack these antigens presumptively have a defect (e.g., mutation or a deletion) in the retinoblastoma gene.

### Deposits

Plasmids p2AR3.8 and p2AR0.9 were deposited on July 17, 1987 with the American Type Culture Collection, Rockville, Maryland, and assigned ATCC accession numbers 40,241 and 40,242, respectively.

The Applicants represent the ATCC is a depository affording permanence of the deposit and ready accessibility thereto by the public if a patent is granted. All restrictions on the availability to the public of the material so deposited will be irrevocably removed upon the granting of a patent. The material will be available during the pendancy of the patent application to one determined by the Commissioner to be entitled thereto under 37 CFR 1.14 and 35 USC 122. The deposited material will be maintained with all the care necessary to keep it viable and uncontaminated for a period of at least five years after the most recent request for the furnishing of a sample of the deposited microorganism, and in any case, for a period of at least thirty (30) years after the date of deposit or for the enforceable life of the patent, whichever period is longer. Applicant acknowledges its duty to replace the deposit should the depository be unable to furnish a sample when requested due to the condition of the deposit.

Other embodiments are within the following claims.

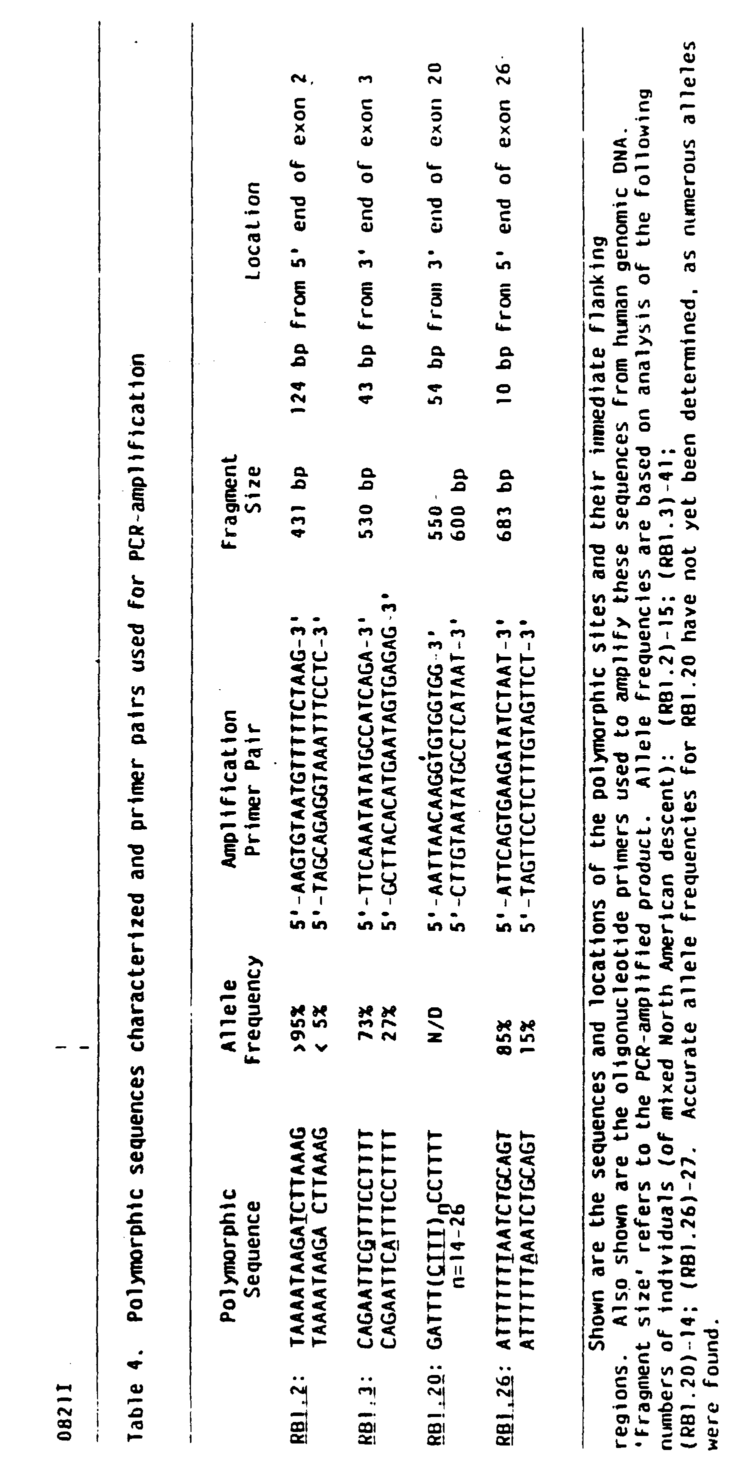

**Table 3**

| DNA sequence polymorphisms detected by direct sequencing | | |
|---|---|---|
| | Base Pairs Screened | Polymorphisms |
| Introns | 2072 | 4 |
| Exons | 1640 | 0 |
| Totals | 3712 | 4 |
| DNA sequence polymorphisms found by direct sequencing of 13 separate PCR-amplified regions from the human retionoblastoma locus. DNA samples from a minimum of 9 unrelated individuals were examined for all bases screened. Bases that could not be scored unambiguously were excluded from this tabulation. | | |

## Claims

1. A method of diagnosing predisposition of a human patient to retinoblastoma, said method comprising the step of detecting, in the retinoblastoma gene of said patient, a genetic polymorphism selected from the group consisting of:
(a) a restriction fragment length polymorphism (RFLP) in the genomic retinoblastoma gene of said patient;
(b) an altered recognition sequence of a restriction endonuclease selected from the group consisting of RsaI, KpnI, XbaI, MboII, and TthlllI;
(c) variability in the number of tandem repeats of at least a substantial portion of the nucleic acid sequence set out below, namely: 
TG          A                                    AG
CGTGTTGAA ( A ) ACAC ( C ) TCCCCAATGCTGGAGGTGAGGTTTGGT ( AGAAG ) ATGACT
            G
 where the brackets "(" and ")" denote regions of variability within the repeat unit and the bases underlined above and below the bracketed regions denote possible alternate bases for those regions of variability;
(d) variability in the number of tandem repeats of the nucleic acid sequence GGGNNGTGGGG, where N is A, T, C, or G.

## Patentansprüche

1. Verfahren zum Diagnostizieren einer Veranlagung zur Ausbildung eines Retinoblastoms beim Menschen, wobei dieses Verfahren den Schritt Entdecken eines genetischen Polymorphismus, ausgewählt aus der Gruppe bestehend aus:
(a) einem Restriktionsfragment-Längenpolymorphismus (RFLP) im genomischen Retionoblastom-Gen des Patienten;
(b) einer veränderten Erkennungssequenz einer Restriktionsendonuclease, ausgewählt aus der Gruppe bestehend aus Rsa I, Kpn I, Xba I, Mbo II und Tth 111 I;
(c) Variabilität in der Anzahl von Tandemwiederholungen von mindestens einem wesentlichen Abschnitt der nachfolgend dargestellten Nucleinsäuresequenz, nämlich: 
TG                    A                                                                                    AG
CGTGTTGAA (  A) ACAC (C) TCCCCAATGCTGGAGGTGAGGTTTGGT (AGAAG) ATGACT
                    G
 wobei die Klammern "(" und ")" die Variabilitätsregionen innerhalb der Wiederholungseinheit kennzeichnen und die oberhalb und unterhalb der in Klammern gesetzten Regionen unterstrichenen Basen mögliche alternative Basen für diese Variabilitätsregionen kennzeichnen;
(d) Variabilität in der Anzahl von Tandemwiederholungen der Nucleinsäuresequenz GGGNNGTGGGG, worin N für A, T, C oder G steht;
im Retinoblastom-Gen dieses Patienten umfaßt.

## Revendications

1. Procédé pour diagnostiquer une prédisposition d'un patient humain au rétinoblastome, ledit procédé comprenant l'étape de détection, dans le gène du rétinoblastome dudit patient, d'un polymorphisme génétique choisi dans le groupe consistant en :
(a) un polymorphisme de taille des fragments de restriction (PTFR) dans le gêne du rétinoblastome génomique dudit patient ;
(b) une séquence de reconnaissance modifiée d'une endonucléase de restriction choisie dans le groupe consistant en RsaI, KpnI, XbaI, MboII et Tth111I ;
(c) une variabilité dans le nombre de répétitions en tandem d'au moins une partie sensible de la séquence d'acide nucléique présentée ci-dessous, à savoir : 
TG A AG
CGTGTTGAA( A)ACAC(C)TCCCCAATGCTGGAGGTGAGGTTTGGT(AGAAG)ATGACT
G
 où les parenthèses "("et")" désignent des régions de variabilité dans l'unité répétée et les bases soulignées au-dessus et au-dessous des régions entre parenthèses désignent d'autres bases possibles pour ces régions de variabilité,
(d) une variabilité dans le nombre de répétitions en tandem de la séquence d'acide nucléique GGGNNGTGGGG, où N est A, T, C ou G.
